# EUROPEAN PATENT SPECIFICATION

(11) **EP 4 613 206 B1**
(45) Date of publication and mention of the grant of the patent: **11.11.2026**
(21) Application number: 25159967.6
(22) Date of filing: 25.02.2025
(51) Int. Cl.: A61B 8/00, G06T 7/73

(54) **ULTRASOUND DIAGNOSTIC APPARATUS AND METHOD OF CONTROLLING ULTRASOUND DIAGNOSTIC APPARATUS**
ULTRASCHALLDIAGNOSEVORRICHTUNG UND VERFAHREN ZUR STEUERUNG DER ULTRASCHALLDIAGNOSEVORRICHTUNG
APPAREIL DE DIAGNOSTIC À ULTRASONS ET PROCÉDÉ DE COMMANDE D'APPAREIL DE DIAGNOSTIC À ULTRASONS

(30) Priority: 04.03.2024 JP 2024032011
(43) Date of publication of application: 10.09.2025
(73) Proprietor: FUJIFILM Corporation, Tokyo 106-8620 (JP)
(72) Inventor: Matsumoto, Tsuyoshi, Kanagawa, 258-8538 (JP)
(74) Representative: Klunker IP Patentanwälte PartG mbB

(56) References cited:
- EP-B1- 2 934 328
- EP-B1- 3 013 242
- US-A1- 2021 315 545

## Description

### BACKGROUND OF THE INVENTION

### 1. Field of the Invention

The present invention relates to an ultrasound diagnostic apparatus used for an examination of an abdomen of a subject, and a method of controlling an ultrasound diagnostic apparatus.

### 2. Description of the Related Art

In the related art, an ultrasound image representing a tomogram of a subject is captured by using a so-called ultrasound diagnostic apparatus, thereby examining an inside of the subject. In this case, a user of the ultrasound diagnostic apparatus usually moves an ultrasound probe to a position on the subject at which a target part can be imaged while checking the captured ultrasound image, but it may be difficult for the user who has a low skill level of an examination using the ultrasound diagnostic apparatus to determine which part of the subject is imaged even in a case in which the ultrasound image is checked and as a result, it may be not possible to move the ultrasound probe to a position for imaging the target part.

Therefore, for example, as disclosed in JP2021-522956A, a technology of guiding a user to a position of an ultrasound probe on a subject at which a cross section for appropriately examining a target part can be imaged, by analyzing an ultrasound image has been developed.

EP 3 013 242 B1 discloses techniques for anatomically intelligent echocardiography for point-of-care. An apparatus includes an imaging probe and is configured for dynamically arranging presentation of visual feedback for guiding manual adjustment, via the probe, of a location, and orientation, associated with the probe. The arranging is selectively based on comparisons between fields of view of the probe and respective results of segmenting image data acquired via the probe. In an embodiment, the feedback does not include a grayscale depiction of the image data. Coordinate system transformations corresponding to respective comparisons may be computed. The selecting may be based upon and dynamically responsive to content of imaging being dynamically acquired via the probe. EP 2 934 328 B1 discloses an anatomically intelligent echocardiography for point-of-care. US 2021/315545 A1 discloses an ultrasonic diagnostic apparatus.

### SUMMARY OF THE INVENTION

Since a shape of an abdomen of the subject is usually changed in accordance with a breathing state and a posture of the subject, in a case in which the abdomen of the subject is examined by the ultrasound diagnostic apparatus, an image pattern of the ultrasound image to be captured is changed in accordance with the breathing state and the posture of the subject even though a position and an angle of the ultrasound probe on a body surface of the subject is not changed. In addition, in a case in which the abdomen of the subject is examined by the ultrasound diagnostic apparatus, the ultrasound image may be captured while pressing the ultrasound probe against the body surface of the subject to apply a pressure, and the image pattern of the ultrasound image to be captured is also changed in accordance with a pressing amount of the ultrasound probe in this case. For example, even in a case in which the technology of JP2021-522956A is used, a user who has a low skill level for the examination of the abdomen of the subject may not be able to appropriately manage the breathing state of the subject, the posture of the subject, and the pressing amount of the ultrasound probe, and may not be able to visualize a recommended cross section, which is a cross section recommended in the examination.

The present invention has been made in order to solve such a problem in the related art, and an object of the present invention is to provide an ultrasound diagnostic apparatus that enables a user to appropriately visualize a recommended cross section regardless of a skill level, and a method of controlling an ultrasound diagnostic apparatus.

With the following configuration, the above-described object can be achieved.
[1] In one aspect, there is provided an ultrasound diagnostic apparatus according to claim 1 of the appended claims.
[2] The ultrasound diagnostic apparatus according to [1], further comprising: a cause specifying unit that specifies a cause of the examination being inappropriate from among the breathing state of the subject, the pressing amount of the ultrasound probe against the subject, and the posture of the subject by performing the image analysis on the ultrasound image, in which the instruction unit instructs the user to change at least one of the breathing state of the subject, the pressing amount of the ultrasound probe against the subject, or the posture of the subject, which is specified as the cause by the cause specifying unit.
[3] The ultrasound diagnostic apparatus according to [2], further comprising: an appropriateness degree determination unit that calculates at least one of a current level of the breathing state of the subject, a current level of the pressing amount of the ultrasound probe, or a current posture of the subject as an indicator related to the cause by performing the image analysis on the ultrasound image, and determines an appropriateness degree of the calculated indicator.
[4] The ultrasound diagnostic apparatus according to [3], in which the instruction unit instructs the user on at least one of an amount of change in the level of the breathing state, an amount of change in the level of the pressing amount of the ultrasound probe, or a target posture of the subject, which is required to determine that the examination is appropriate, based on the indicator and the appropriateness degree that are obtained by the appropriateness degree determination unit.
[5] The ultrasound diagnostic apparatus according to any one of [1] to [4], in which the instruction unit instructs the user to change at least one of the breathing state of the subject, the pressing amount of the ultrasound probe against the subject, or the posture of the subject at an indicator position and an indicator posture angle of the ultrasound probe for imaging an indicator cross section different from the recommended cross section.
[6] A method of controlling an ultrasound diagnostic apparatus, the method comprising: detecting a position and a posture angle of an ultrasound probe; acquiring an ultrasound image of an inside of a subject by transmitting and receiving an ultrasound beam by using the ultrasound probe; recognizing a predetermined recommended cross section for a part of the subject by performing image analysis on the ultrasound image; determining that an examination is inappropriate in a case in which the recommended cross section is not recognized in a predetermined positional/angular range including a recommended position and a recommended posture angle of the ultrasound probe corresponding to the recommended cross section with reference to the position and the posture angle of the ultrasound probe, which are detected; and instructing a user to change at least one of a breathing state of the subject, a pressing amount of the ultrasound probe against the subject, or a posture of the subject in a case in which it is determined that the examination is inappropriate.

The aspect of the present invention relates to the ultrasound diagnostic apparatus comprising: the ultrasound probe; the position/posture detection unit that detects the position and the posture angle of the ultrasound probe; the image acquisition unit that acquires the ultrasound image of the inside of the subject by transmitting and receiving the ultrasound beam by using the ultrasound probe; the recommended cross section recognition unit that recognizes the predetermined recommended cross section for the part of the subject by performing the image analysis on the ultrasound image acquired by the image acquisition unit; the examination determination unit that determines that the examination is inappropriate in a case in which the recommended cross section is not recognized by the recommended cross section recognition unit in the predetermined positional/angular range including the recommended position and the recommended posture angle of the ultrasound probe corresponding to the recommended cross section with reference to the position and the posture angle of the ultrasound probe, which are detected by the position/posture detection unit; and the instruction unit that instructs the user to change at least one of the breathing state of the subject, the pressing amount of the ultrasound probe against the subject, or the posture of the subject in a case in which the examination determination unit determines that the examination is inappropriate, so that the user appropriately visualizes the recommended cross section regardless of the skill level.

### BRIEF DESCRIPTION OF THE DRAWINGS

Fig. 1 is a block diagram showing a configuration of an ultrasound diagnostic apparatus according to Embodiment 1 of the present invention.
Fig. 2 is a block diagram showing a configuration of a transceiver circuit according to Embodiment 1 of the present invention.
Fig. 3 is a block diagram showing a configuration of an image generation unit according to Embodiment 1 of the present invention.
Fig. 4 is a diagram showing an example of an instruction to a user according to Embodiment 1 of the present invention.
Fig. 5 is a flowchart showing an operation of the ultrasound diagnostic apparatus according to Embodiment 1 of the present invention.
Fig. 6 is a block diagram showing a configuration of an ultrasound diagnostic apparatus according to Embodiment 2 of the present invention.
Fig. 7 is a block diagram showing a configuration of an ultrasound diagnostic apparatus according to Embodiment 3 of the present invention.
Fig. 8 is a block diagram showing a configuration of an ultrasound diagnostic apparatus according to Embodiment 4 of the present invention.
Fig. 9 is a diagram showing a first example of an instruction to a user according to Embodiment 4 of the present invention.
Fig. 10 is a diagram showing a second example of the instruction to the user according to Embodiment 4 of the present invention.

### DESCRIPTION OF THE PREFERRED EMBODIMENTS

Hereinafter, embodiments of the present invention will be described based on the accompanying drawings.

The configuration requirements are described later based on a representative embodiment of the present invention, but the present invention is not limited to such an embodiment.

In the present specification, a numerical range represented by "to" means a range including numerical values described before and after "to" as a lower limit value and an upper limit value.

In the present specification, "the same" includes an error range generally allowed in the technical field.

### Embodiment 1

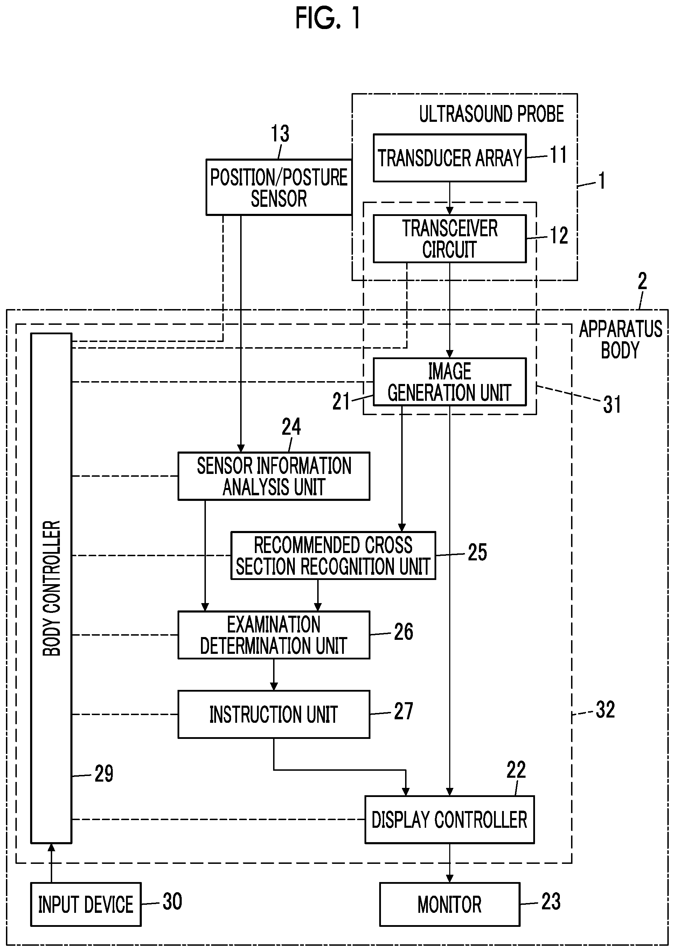

Fig. 1 shows a configuration of an ultrasound diagnostic apparatus according to Embodiment 1 of the present invention. The ultrasound diagnostic apparatus comprises an ultrasound probe 1, and an apparatus body 2 connected to the ultrasound probe 1. The ultrasound probe 1 and the apparatus body 2 are connected to each other via so-called wired communication or so-called wireless communication.

The ultrasound probe 1 includes a transducer array 11. A transceiver circuit 12 is connected to the transducer array 11. The ultrasound probe 1 includes a position/posture sensor 13. The position/posture sensor 13 may be incorporated in the ultrasound probe 1 or attached to a housing of the ultrasound probe 1. In addition, in a case in which a sensor device that measures the ultrasound probe 1 from the outside, such as a so-called optical sensor, is used as the position/posture sensor 13, the position/posture sensor 13 may be disposed at a position away from the ultrasound probe 1.

The apparatus body 2 includes an image generation unit 21 connected to the transceiver circuit 12 of the ultrasound probe 1. A display controller 22 and a monitor 23 are sequentially connected to the image generation unit 21. In addition, the apparatus body 2 includes a sensor information analysis unit 24 that is connected to the position/posture sensor 13 of the ultrasound probe 1. A recommended cross section recognition unit 25 is connected to the image generation unit 21, and an examination determination unit 26 is connected to the sensor information analysis unit 24 and the recommended cross section recognition unit 25. An instruction unit 27 is connected to the examination determination unit 26. The instruction unit 27 is connected to the display controller 22. In addition, a body controller 29 is connected to the transceiver circuit 12, the position/posture sensor 13, the image generation unit 21, the display controller 22, the sensor information analysis unit 24, the recommended cross section recognition unit 25, the examination determination unit 26, and the instruction unit 27. An input device 30 is connected to the body controller 29.

The transceiver circuit 12 and the image generation unit 21 constitute an image acquisition unit 31. In addition, the image generation unit 21, the display controller 22, the sensor information analysis unit 24, the recommended cross section recognition unit 25, the examination determination unit 26, the instruction unit 27, and the body controller 29 constitute a processor 32 for the apparatus body 2.

The transducer array 11 of the ultrasound probe 1 includes a plurality of ultrasound transducers arranged one-dimensionally or two-dimensionally. In response to a drive signal supplied from the transceiver circuit 12, each of the ultrasound transducers transmits ultrasound and receives an ultrasound echo from a subject to output a signal based on the ultrasound echo. For example, each ultrasound transducer is configured by forming electrodes at both ends of a piezoelectric material consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), and the like.

The image acquisition unit 31 configured by the transceiver circuit 12 and the image generation unit 21 acquires an ultrasound image of an inside of the subject by transmitting and receiving an ultrasound beam by using the ultrasound probe 1.

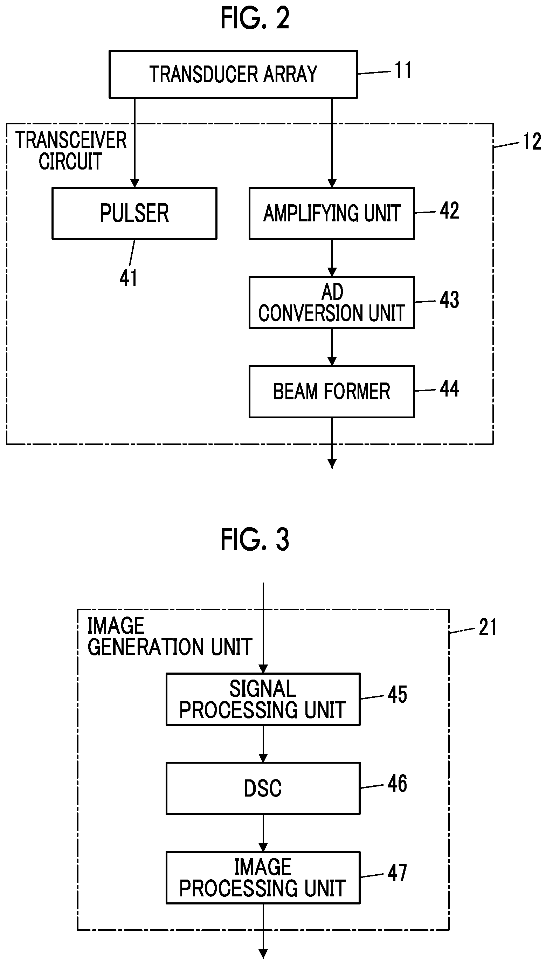

Under the control of the body controller 29, the transceiver circuit 12 causes the transducer array 11 to transmit the ultrasound and generates a sound ray signal based on a reception signal acquired by the transducer array 11. As shown in Fig. 2, the transceiver circuit 12 includes a pulser 41 connected to the transducer array 11, and an amplifying unit 42, an analog-to-digital (AD) conversion unit 43, and a beam former 44 that are sequentially connected in series to the transducer array 11.

The pulser 41 includes, for example, a plurality of pulse generators, and the pulser 41 adjusts an amount of delay of each drive signal such that the ultrasound transmitted from the plurality of ultrasound transducers of the transducer array 11 form the ultrasound beam, based on a transmission delay pattern selected in accordance with a control signal from the body controller 29, and supplies the adjusted signal to the plurality of ultrasound transducers. In this way, in a case in which a pulsed or continuous wave-like voltage is applied to the electrodes of the ultrasound transducer of the transducer array 11, the piezoelectric material expands and contracts to generate pulsed or continuous wave-like ultrasound from each of the ultrasound transducers, whereby the ultrasound beam is formed from the combined wave of the ultrasound.

The transmitted ultrasound beam is, for example, reflected in a target such as a part of the subject and propagates toward the transducer array 11 of the ultrasound probe 1. The ultrasound echo propagating toward the transducer array 11 in this way is received by each of the ultrasound transducers constituting the transducer array 11. In this case, each of the ultrasound transducers constituting the transducer array 11 receives the propagating ultrasound echo to expand and contract to generate the reception signal, which is an electrical signal, and outputs these reception signals to the amplifying unit 42.

The amplifying unit 42 amplifies the signal input from each of the ultrasound transducers constituting the transducer array 11 and transmits the amplified signal to the AD conversion unit 43. The AD conversion unit 43 converts the signal transmitted from the amplifying unit 42 into digital reception data. The beam former 44 performs so-called reception focus processing by applying and adding the delay to each reception data received from the AD conversion unit 43. By this reception focus processing, each reception data converted by the AD conversion unit 43 is phase-added, and the sound ray signal in which the focus of the ultrasound echo is narrowed down is acquired.

As shown in Fig. 3, the image generation unit 21 has a configuration in which a signal processing unit 45, a digital scan converter (DSC) 46, and an image processing unit 47 are sequentially connected in series to each other.

The signal processing unit 45 generates a B-mode image signal, which is tomographic image information related to tissues inside the subject, by performing, on the sound ray signal received from the transceiver circuit 12, correction of the attenuation due to a distance in accordance with a depth of a reflection position of the ultrasound by using a sound velocity value set by the body controller 29 and then performing envelope detection processing.

The DSC 46 converts (raster-converts) the B-mode image signal generated by the signal processing unit 45 into the image signal in accordance with a normal television signal scanning method.

The image processing unit 47 performs various types of necessary image processing such as gradation processing on the B-mode image signal input from the DSC 46, and then sends the B-mode image signal to the display controller 22 and the recommended cross section recognition unit 25. Hereinafter, the B-mode image signal that is image-processed by the image processing unit 47 will be referred to as an ultrasound image.

The position/posture sensor 13 is a sensor device that acquires a signal representing the position and the posture angle of the ultrasound probe 1 under the control of the body controller 29. The position of the ultrasound probe 1 represents a position of the ultrasound probe 1 in a three-dimensional space. The posture angle of the ultrasound probe 1 represents an inclined angle and a rotation angle of the ultrasound probe 1 in the three-dimensional space. The position/posture sensor 13 can include, for example, at least one of a so-called inertial sensor, a magnetic sensor, an optical sensor, or an optical camera. The inertial sensor can include, for example, at least one of a so-called acceleration sensor or a gyro sensor.

The sensor information analysis unit 24 analyzes the signal transmitted from the position/posture sensor 13 to acquire information representing the position and the posture angle of the ultrasound probe 1. The sensor information analysis unit 24 can acquire the information representing the position and the posture angle of the ultrasound probe 1 by using, for example, a trained model in so-called machine learning, which has learned a large amount of information on a relationship between the signal obtained by the sensor device constituting the position/posture sensor 13 and the position and the posture angle of the ultrasound probe 1. The sensor information analysis unit 24 can acquire, for example, coordinate values of three components along three axes orthogonal to each other in a rectangular coordinate system as the information representing the position of the ultrasound probe 1. Further, the sensor information analysis unit 24 can acquire, for example, a so-called Euler angle defined in a rectangular coordinate system as the information representing the posture angle of the ultrasound probe 1.

The recommended cross section recognition unit 25 performs processing of recognizing a predetermined recommended cross section for a part of the subject by performing image analysis on the ultrasound image generated by the image generation unit 21. Here, the recommended cross section is a cross section in which the examination is recommended for a predetermined part of a specific subject. This recommended cross section can be determined, for example, for each hospital, for each subject, or for each user, or can be determined in accordance with a manual determined by a specialized academic society or the like.

The recommended cross section recognition unit 25 can recognize an image pattern representing the recommended cross section in the ultrasound image by using, for example, a trained model in machine learning, which has learned a relationship between the image patterns of a large number of ultrasound images and the image pattern of the recommended cross section. As the machine learning algorithm, for example, a so-called convolutional neural network (CNN) can be used. In addition, the recommended cross section recognition unit 25 can also recognize the image pattern representing the recommended cross section in the ultrasound image by known image analysis using, for example, a so-called OpenCV (registered trademark) or the like. In addition, the recommended cross section recognition unit 25 can also recognize the image pattern of the recommended cross section in advance and perform so-called template matching processing on the image pattern of the recommended cross section and the image pattern of the acquired ultrasound image, thereby recognizing the image pattern representing the recommended cross section in the ultrasound image.

The examination determination unit 26 stores in advance a recommended position and a recommended posture angle of the ultrasound probe 1 corresponding to the recommended cross section, that is, the recommended position and the recommended posture angle of the ultrasound probe 1 for imaging the recommended cross section, and a predetermined positional/angular range including the recommended position and the recommended posture angle, and determines that the examination for the subject is inappropriate in a case in which the recommended cross section is not recognized by the recommended cross section recognition unit 25 in the predetermined positional/angular range, with reference to the position and the posture angle of the ultrasound probe 1 acquired by the sensor information analysis unit 24. In addition, in a case in which the recommended cross section is recognized by the recommended cross section recognition unit 25 in the predetermined positional/angular range, the examination determination unit 26 determines that the examination for the subject is appropriate. It should be noted that examination determination unit 26 can determine whether the examination is appropriate or inappropriate for the ultrasound image captured in a positional/angular range other than the predetermined positional/angular range.

Here, it is preferable that the positional/angular range is set in accordance with the presence or absence of the bone present around the recommended cross section, the thickness of the fat, the influence degree of the gas in the intestinal tract, and the like, and for example, in a case in which an axis perpendicular to the body surface of the subject is defined as a Z axis and two axes orthogonal to the Z axis are defined as an X axis and a Y axis, the positional/angular range can be set as a range including a position range having a width of several cm along each of the X axis, the Y axis, and the Z axis with the recommended position as a center, and an angle range of 90 degrees with the recommended posture angle as a center.

Since a shape of an abdomen of the subject is usually changed in accordance with a breathing state and a posture of the subject, in a case in which the abdomen of the subject is examined by the ultrasound diagnostic apparatus, the image pattern of the ultrasound image to be captured is changed in accordance with the breathing state and the posture of the subject even though the position and the angle of the ultrasound probe 1 on the body surface of the subject is not changed. In addition, in a case in which the abdomen of the subject is examined by the ultrasound diagnostic apparatus, the ultrasound image may be captured while pressing the ultrasound probe 1 against the body surface of the subject to apply a pressure to the abdomen, and the image pattern of the ultrasound image to be captured is also changed in accordance with an pressing amount of the ultrasound probe 1 in this case. The user who has a low skill level for the examination of the abdomen of the subject may not be able to appropriately manage the breathing state of the subject, the posture of the subject, and the pressing amount of the ultrasound probe 1, and may not be able to appropriately visualize the recommended cross section.

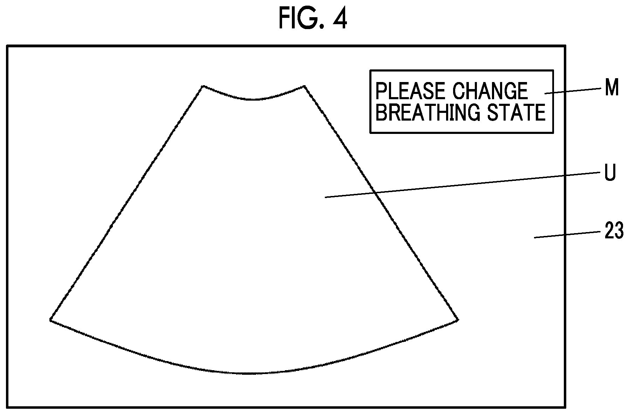

Therefore, in a case in which the examination determination unit 26 determines that the examination is inappropriate, the instruction unit 27 instructs the user to change at least one of the breathing state of the subject, the pressing amount of the ultrasound probe 1 against the subject, or the posture of the subject. For example, as shown in Fig. 4, the instruction unit 27 can instruct the user to appropriately visualize the recommended cross section by displaying a message M such as "Please change the breathing state" on the monitor 23. In the example of Fig. 4, an ultrasound image U currently being captured and the message M are displayed together on the monitor 23. The instruction unit 27 can also display, for example, the message M such as "Please change the pressing amount of the probe" or "Please change the posture of the subject" on the monitor 23.

The user can check the instruction from the instruction unit 27 to understand that the cause of the failure to appropriately visualize the recommended cross section is at least one of the breathing state of the subject, the pressing amount of the ultrasound probe 1 against the subject, or the posture of the subject, to appropriately and clearly visualize the recommended cross section regardless of the skill level by changing at least one of the breathing state of the subject, the pressing amount of the ultrasound probe 1 against the subject, or the posture of the subject.

Under the control of the body controller 29, the display controller 22 performs predetermined processing on the ultrasound image U sent from the image generation unit 21, the information indicating the instruction to the user issued by the instruction unit 27, and the like, and displays the ultrasound image U and the information on the monitor 23.

The monitor 23 is a monitor for displaying the ultrasound image U, the instruction to the user, and the like under the control of the display controller 22, and includes a display device such as a liquid crystal display (LCD), or an organic electroluminescence (EL) display.

The body controller 29 controls each unit of the apparatus body 2, the transceiver circuit 12 of the ultrasound probe 1, and the position/posture sensor 13 based on a control program and the like stored in advance.

The input device 30 is an input device for the user to perform an input operation, and is configured by, for example, a device such as a keyboard, a mouse, a trackball, a touchpad, and a touch sensor disposed in a state of being superimposed on the monitor 23.

It should be noted that the processor 32 including the image generation unit 21, the display controller 22, the sensor information analysis unit 24, the recommended cross section recognition unit 25, the examination determination unit 26, the instruction unit 27, and the body controller 29 is configured by a central processing unit (CPU) and the control program for causing the CPU to execute various types of processing, but the processor 32 may be configured by a field programmable gate array (FPGA), a digital signal processor (DSP), an application specific integrated circuit (ASIC), a graphics processing unit (GPU), or other integrated circuits (IC), or may be configured by a combination thereof.

In addition, the image generation unit 21, the display controller 22, the sensor information analysis unit 24, the recommended cross section recognition unit 25, the examination determination unit 26, the instruction unit 27, and the body controller 29 can also be configured by being integrated partially or entirely into one CPU or the like.

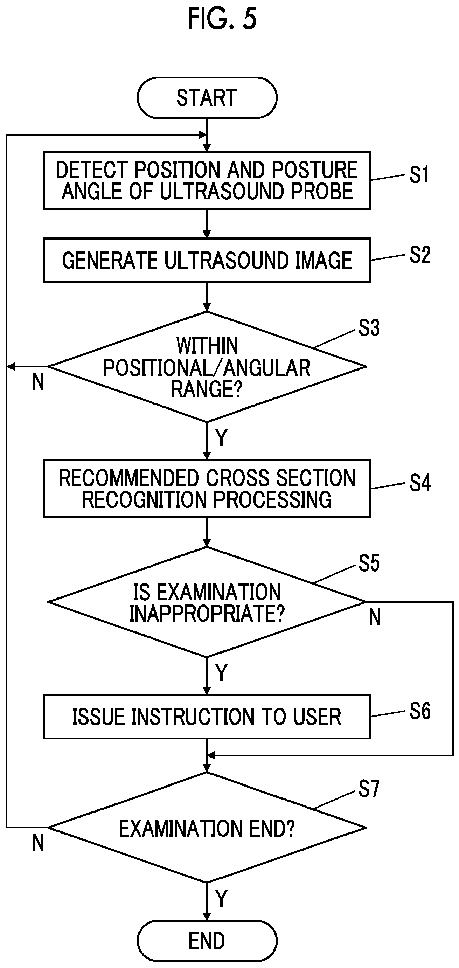

Next, an operation of the ultrasound diagnostic apparatus according to Embodiment 1 will be described with reference to the flowchart shown in Fig. 5. Hereinafter, a case will be described in which a specific organ in the abdomen of the subject is selected as the examination target of the subject, and the recommended cross section corresponding to the selected organ and the predetermined positional/angular range corresponding to the recommended cross section are stored in the examination determination unit 26. It should be noted that organ as the examination target can be selected by the user via the input device 30 before the examination starts.

In step S1, the position and the posture angle of the ultrasound probe 1 are detected. In this case, the position/posture sensor 13 acquires a signal representing the current position and the current posture angle of the ultrasound probe 1, and the sensor information analysis unit 24 acquires information representing the position and the posture angle of the ultrasound probe 1 by analyzing the signal acquired by the position/posture sensor 13. The information representing the position and the posture angle of the ultrasound probe 1 acquired in this way is sent to the recommended cross section recognition unit 25 and the examination determination unit 26.

In step S2, the image generation unit 21 generates the ultrasound image U at the current position and the current posture angle of the ultrasound probe 1. In this case, under the control of the body controller 29, the transmission and reception of the ultrasound from the plurality of transducers of the transducer array 11 are started in accordance with the drive signal from the pulser 41 of the transceiver circuit 12 of the ultrasound probe 1, the ultrasound echo from the subject is received by the plurality of transducers of the transducer array 11, and the reception signal as the analog signal is output to the amplifying unit 42, is amplified, and then is subjected to the AD conversion via the AD conversion unit 43 to acquire the reception data.

The reception focus processing is performed on the reception data by the beam former 44, and the sound ray signal generated by the reception focusing processing is sent to the image generation unit 21 of the apparatus body 2, thereby the ultrasound image U representing the tomographic image information of the subject is generated by the image generation unit 21. In this case, the signal processing unit 45 of the image generation unit 21 performs the correction of the attenuation in accordance with the depth of the reflection position of the ultrasound and the envelope detection processing on the sound ray signal, the DSC 46 performs the conversion into the image signal in accordance with the normal television signal scanning method, and the image processing unit 47 performs various types of necessary image processing such as gradation processing. The ultrasound image U generated in step S2 in this way is displayed on the monitor 23 via the display controller 22 and is sent to the recommended cross section recognition unit 25.

In step S3, the examination determination unit 26 determines whether or not the position and the posture angle of the ultrasound probe 1 detected in step S1 are within the predetermined positional/angular range corresponding to the recommended cross section selected before the examination. In a case in which it is determined in step S1 that the position and the posture angle of the ultrasound probe 1 detected in step S1 are out of the positional/angular range, the processing returns to step S1, the position and the posture angle of the ultrasound probe 1 are newly detected, the ultrasound image U is acquired in step S2, and it is determined in step S3 whether or not the position and the posture angle of the ultrasound probe 1 are within the predetermined positional/angular range. In this manner, as long as it is determined in step S3 that the position and the posture angle of the ultrasound probe 1 are outside the positional/angular range corresponding to the recommended cross section, the processing of step S1 to step S3 is repeated.

In a case in which it is determined in step S3 that the position and the posture angle of the ultrasound probe 1 are within the positional/angular range corresponding to the recommended cross section, the processing proceeds to step S4. In step S4, the recommended cross section recognition unit 25 performs processing of recognizing the recommended cross section surface shown in the ultrasound image U by performing image analysis on the ultrasound image U acquired in the latest step S2. The recommended cross section recognition unit 25 can recognize the recommended cross section shown in the ultrasound image U by using, for example, a trained model in machine learning, which is constructed by the CNN and has learned a relationship between the image patterns of a large number of ultrasound images U and the image pattern of the recommended cross section. In addition, the recommended cross section recognition unit 25 can also recognize the recommended cross section shown in the ultrasound image U by known image analysis using, for example, a so-called OpenCV (registered trademark) or the like.

In step S5, the examination determination unit 26 determines whether or not the examination for the subject is inappropriate based on the determination in step S3 and the result of the recognition processing of the recommended cross section in step S4. In this case, the examination determination unit 26 can determine that the examination of the subject is inappropriate in a case in which the recommended cross section is not recognized in the predetermined positional/angular range corresponding to the recommended cross section in step S4. In addition, the examination determination unit 26 can determine that the examination of the subject is appropriate in a case in which the recommended cross section is recognized in the predetermined positional/angular range corresponding to the recommended cross section in step S4.

In a case in which it is determined in step S5 that the examination for the subject is inappropriate, the processing proceeds to step S6. In step S6, the instruction unit 27 instructs the user to change at least one of the breathing state of the subject, the pressing amount of the ultrasound probe 1 against the subject, or the posture of the subject. For example, as shown in Fig. 4, the instruction unit 27 can instruct the user to appropriately visualize the recommended cross section by displaying the message M such as "Please change the breathing state" on the monitor 23.

Here, in general, in a case in which the abdomen of the subject is examined by using the ultrasound diagnostic apparatus, the image pattern visualized in the ultrasound image U is changed in accordance with the breathing state of the subject, the pressing amount of the ultrasound probe 1 against the subject, and the posture of the subject. Therefore, in order to appropriately visualize the recommended cross section, it is required to appropriately manage the breathing state of the subject, the pressing amount of the ultrasound probe 1 against the subject, or the posture of the subject.

The user can check the instruction from the instruction unit 27 in step S6 to understand that the examination is inappropriate due to the breathing state of the subject, the pressing amount of the ultrasound probe 1 against the subject, or the posture of the subject, so that the user can appropriately visualize the recommended cross section regardless of the skill level by changing at least one of the breathing state of the subject, the pressing amount of the ultrasound probe 1 against the subject, or the posture of the subject in accordance with the instruction.

In a case in which the processing of step S6 is completed in this way, the processing proceeds to step S7. In addition, in a case in which it is determined in step S5 that the examination for the subject is appropriate, step S6 is skipped, and the processing proceeds to step S7.

In step S7, the body controller 29 determines whether or not to end the examination of the subject. The body controller 29 can determine to end the examination in a case in which the user inputs an instruction to end the examination via the input device 30, for example, because the examination can be appropriately performed on the subject, and determine to continue the examination in a case in which the instruction to end the examination is not particularly input. In a case in which it is determined in step S7 to continue the examination, the processing returns to step S1, and then the processing of step S2 to step S7 is performed again. In a case in which it is determined to end the examination in step S7, the operation of the ultrasound diagnostic apparatus according to the flowchart of Fig. 5 is completed.

As described above, with the ultrasound diagnostic apparatus according to Embodiment 1, the examination determination unit 26 determines that the examination is inappropriate in a case in which the recommended cross section is not recognized by the recommended cross section recognition unit 25 in the predetermined positional/angular range including the recommended position and the recommended posture angle of the ultrasound probe 1 corresponding to the recommended cross section with reference to the position and the posture angle of the ultrasound probe 1, which are acquired by the sensor information analysis unit 24, and the instruction unit 27 instructs the user to change at least one of the breathing state of the subject, the pressing amount of the ultrasound probe 1 against the subject, or the posture of the subject in a case in which the examination determination unit 26 determines that the examination is inappropriate, so that the user can appropriately visualize the recommended cross section regardless of the skill level.

It should be noted that the description is made in which the ultrasound probe 1 comprises the transceiver circuit 12, but the apparatus body 2 may comprise the transceiver circuit 12.

In addition, the description has been made in which the apparatus body 2 comprises the image generation unit 21, but the ultrasound probe 1 may comprise the image generation unit 21.

In addition, the apparatus body 2 may be a so-called stationary type, a portable type that is easily carried, or a so-called hand-held type configured by, for example, a smartphone or tablet computer. As described above, the type of the device constituting the apparatus body 2 is not particularly limited.

In addition, although the description is made in which the examination determination unit 26 stores the recommended cross section, in a case in which the ultrasound diagnostic apparatus comprises a memory (not shown), the image pattern of the recommended cross section, the recommended position and the recommended posture angle of the ultrasound probe 1 corresponding to the recommended cross section, and the information on the predetermined positional/angular range including the recommended position and the recommended posture angle can also be stored in the memory. The examination determination unit 26 can read out these pieces of information stored in the memory, to use these pieces of information.

Here, as the memory, for example, a recording medium such as a flash memory, a hard disk drive (HDD), a solid-state drive (SSD), a flexible disk (FD), a magneto-optical disk (MO disk), a magnetic tape (MT), a random-access memory (RAM), a compact disc (CD), a digital versatile disc (DVD), a secure digital card (SD card), or a universal serial bus memory (USB memory) can be used.

Although the description is made in which the instruction unit 27 issues the instruction to the user by displaying the message M on the monitor 23, for example, in a case in which the ultrasound diagnostic apparatus comprises a speaker (not shown), the instruction unit 27 can also issues the instruction to the user by voice via the speaker. In particular, in a case in which the instruction unit 27 issues the instruction on the breathing state of the subject or the posture of the subject by voice, the subject can understand the operation to be performed from now on, so that the instruction unit 27 can also issue an instruction to the subject in addition to the instruction to the user.

In addition, in the examination of the abdomen of the subject using the ultrasound diagnostic apparatus, in a case in which the user has a low skill level changes at least one of the breathing state of the subject, the pressing amount of the ultrasound probe 1, or the posture of the subject in the vicinity of the position and the posture angle of the ultrasound probe 1 that can image the recommended cross section, it may be difficult to read from the ultrasound image U whether or not the breathing state of the subject, the pressing amount of the ultrasound probe 1, or the posture of the subject is appropriately changed. Therefore, for example, the instruction unit 27 can store in advance an indicator cross section showing the characteristic structure present in the vicinity of the recommended cross section and an indicator position and an indicator posture angle of the ultrasound probe 1 for imaging the indicator cross section, and then instruct the user to change at least one of the breathing state of the subject, the pressing amount of the ultrasound probe 1, or the posture of the subject at the indicator position and the indicator posture angle after guiding the scanning with the ultrasound probe 1 toward the indicator position and the indicator posture angle.

Since the indicator cross section shows the characteristic structure, it is easy to understand a change in a degree of clarity in accordance with the changes in the breathing state of the subject, the pressing amount of the ultrasound probe 1, and the posture of the subject. Therefore, the user can appropriately change the breathing state of the subject, the pressing amount of the ultrasound probe 1, and the posture of the subject while checking the visualized indicator cross section. The user can appropriately visualize the recommended cross section by visualizing the recommended cross section as it is after adjusting at least one of the breathing state of the subject, the pressing amount of the ultrasound probe 1, or the posture of the subject in this manner.

In addition, the description is made in which the user designates the organ as the examination target in advance before the examination is started, but, for example, a part of the organ and a scanning location or a scanning method thereof, such as a so-called right subcostal scanning of the prehepatic upper segment, can also be designated in addition to the name of the organ to be designated as the examination target is not limited.

The ultrasound diagnostic apparatus can analyze the captured ultrasound image U to specify the examination target instead of manually designating the examination target by the user. For example, the apparatus body 2 can comprise an examination target specifying unit (not shown) that specifies the examination target from the ultrasound image U. The examination target specifying unit is connected to, for example, the image generation unit 21, the recommended cross section recognition unit 25, the examination determination unit 26, and the body controller 29. The examination target specifying unit can detect a structure in the ultrasound image U by using, for example, a trained model in machine learning, which has learned a large number of ultrasound images U of a plurality of examination targets, and an image analysis technique using a feature amount such as template matching, AdaBoost, a support-vector machine (SVM), or a scale invariant feature transform (SIFT), and can specify the type of the structure. In this case, for example, the body controller 29 can specify the examination target based on the type of the structure specified by the examination target specifying unit.

In addition, in the flowchart of Fig. 5, after determining whether or not the position and the posture angle of the ultrasound probe 1 are within the predetermined positional/angular range in step S3, it is determined in step S5 whether or not the recommended cross section can be recognized, to determine whether or not the examination is inappropriate, but it can be determined whether or not the examination is inappropriate by determining whether or not the recommended cross section can be recognized, and then determining whether or not the position and the posture angle of the ultrasound probe 1 are within the predetermined positional/angular range for the ultrasound image U in which the recommended cross section can be recognized.

### Embodiment 2

In Embodiment 1, the position/posture detection unit that detects the position and the posture angle of the ultrasound probe 1 is configured by the position/posture sensor 13 and the sensor information analysis unit 24, but, for example, the position and the posture angle of the ultrasound probe 1 can also be detected by performing image analysis on the ultrasound image U.

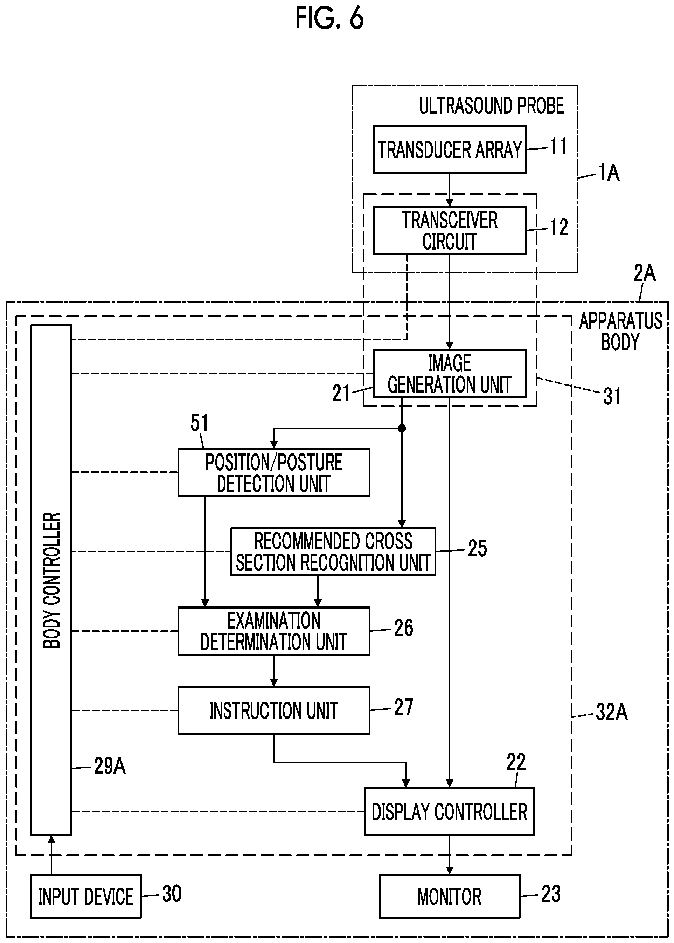

Fig. 6 shows a configuration of an ultrasound diagnostic apparatus according to Embodiment 2. The ultrasound diagnostic apparatus according to Embodiment 2 comprises an ultrasound probe 1A instead of the ultrasound probe 1 and comprises an apparatus body 2A instead of the apparatus body 2, with respect to the ultrasound diagnostic apparatus according to Embodiment 1 shown in Fig. 1.

The ultrasound probe 1A according to Embodiment 2 is obtained by excluding the position/posture sensor 13 from the ultrasound probe 1 according to Embodiment 1.

The apparatus body 2A according to Embodiment 2 comprises a position/posture detection unit 51 instead of the sensor information analysis unit 24 and comprises a body controller 29A instead of the body controller 29, with respect to the apparatus body 2 according to Embodiment 2. In the apparatus body 2A, the position/posture detection unit 51 is connected to the image generation unit 21. The position/posture detection unit 51 is connected to the examination determination unit 26 and the body controller 29A. In addition, the image generation unit 21, the display controller 22, the recommended cross section recognition unit 25, the examination determination unit 26, the instruction unit 27, the body controller 29A, and the position/posture detection unit 51 constitute a processor 32A for the apparatus body 2A.

The position/posture detection unit 51 detects the position and the posture angle of the ultrasound probe 1A in a case in which the ultrasound image U is generated, by performing image analysis on the ultrasound image U generated by the image generation unit 21. The position/posture detection unit 51 can detect the position and the posture angle of the ultrasound probe 1A by using, for example, a trained model in machine learning, which has learned a relationship between a large number of ultrasound images U of the inside of the subject and the position and the posture angle of the ultrasound probe 1A in a case in which the ultrasound images U are captured. In addition, for example, the position/posture detection unit 51 can also store the plurality of ultrasound images U of the inside of the subject associated with the position and the posture angle of the ultrasound probe 1A as templates, and apply a template matching method to the plurality of ultrasound images U stored in advance and the ultrasound image U newly generated by the image generation unit 21, to detect the position and the posture angle of the ultrasound probe 1A.

The position and the posture angle of the ultrasound probe 1A detected by the position/posture detection unit 51 in this way are sent to the examination determination unit 26 and are used for determining whether or not the examination for the subject is inappropriate. In a case in which the examination determination unit 26 determines that the examination on the subject is inappropriate, the instruction unit 27 instructs the user to change at least one of the breathing state of the subject, the pressing amount of the ultrasound probe 1, or the posture of the subject.

As described above, as in the ultrasound diagnostic apparatus according to Embodiment 2, even in a case in which the ultrasound image U is analyzed to detect the position and the posture angle of the ultrasound probe 1A, in a case in which the examination determination unit 26 determines that the examination is inappropriate, the instruction unit 27 instructs the user to change at least one of the breathing state of the subject, the pressing amount of the ultrasound probe 1, or the posture of the subject, so that the user can appropriately visualize the recommended cross section regardless of the skill level.

### Embodiment 3

In Embodiments 1 and 2, the description is made in which the instruction unit 27 instructs the user to change at least one of the breathing state of the subject, the pressing amount of the ultrasound probe 1, or the posture of the subject, but the ultrasound diagnostic apparatus can also specify the cause of the determination that the examination is inappropriate from among the breathing state of the subject, the pressing amount of the ultrasound probe 1, and the posture of the subject.

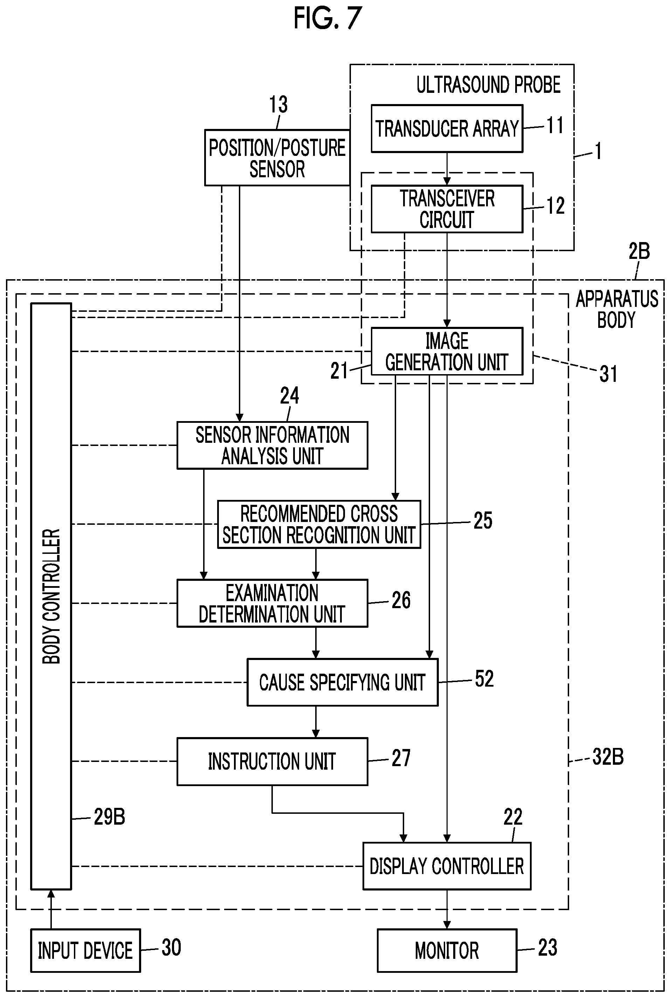

Fig. 7 shows a configuration of an ultrasound diagnostic apparatus according to Embodiment 3. The ultrasound diagnostic apparatus according to Embodiment 3 comprises an apparatus body 2B instead of the apparatus body 2 with respect to the ultrasound diagnostic apparatus according to Embodiment 1 shown in Fig. 1. The apparatus body 2B further comprises a cause specifying unit 52 and comprises a body controller 29B instead of the body controller 29, with respect to the apparatus body 2 according to Embodiment 1.

In the apparatus body 2B, the cause specifying unit 52 is connected to the image generation unit 21 and the examination determination unit 26. The cause specifying unit 52 is connected to the instruction unit 27 and the body controller 29B. In addition, the image generation unit 21, the display controller 22, the sensor information analysis unit 24, the recommended cross section recognition unit 25, the examination determination unit 26, the instruction unit 27, the body controller 29B, and the cause specifying unit 52 constitute a processor 32B for the apparatus body 2B.

In a case in which the examination determination unit 26 determines that the examination on the subject is inappropriate, the cause specifying unit 52 specifies the cause of the inappropriate examination from among the breathing state of the subject, the pressing amount of the ultrasound probe 1 on the subject, and the posture of the subject by performing image analysis on the ultrasound image U generated by the image generation unit 21. The cause specifying unit 52 can specify the cause of the inappropriate examination by using, for example, a trained model in machine learning, which has learned a relationship between a large number of ultrasound images U in a case in which the examination of the subject is inappropriate and the cause of the inappropriate examination.

The instruction unit 27 instructs the user to change at least one of the breathing state of the subject, the pressing amount of the ultrasound probe 1 against the subject, or the posture of the subject, which is specified as the cause of the inappropriate examination by the cause specifying unit 52. The user can appropriately visualize the recommended cross section by changing at least one of the breathing state of the subject, the pressing amount of the ultrasound probe 1 against the subject, or the posture of the subject in accordance with the instruction of the instruction unit 27.

As described above, with the ultrasound diagnostic apparatus according to Embodiment 3, the cause specifying unit 52 specifies the cause of the inappropriate examination of the subject, and the instruction unit 27 instructs the user to change at least one of the breathing state of the subject, the pressing amount of the ultrasound probe 1 against the subject, or the posture of the subject, which is specified as the cause of the inappropriate examination, so that the user can appropriately visualize the recommended cross section regardless of the skill level.

It should be noted that ultrasound diagnostic apparatus according to Embodiment 3 has a configuration in which the cause specifying unit 52 is added to the apparatus body 2 according to Embodiment 1, but a configuration can also be adopted in which the cause specifying unit 52 is added to the apparatus body 2A according to Embodiment 2.

### Embodiment 4

In Embodiment 3, the cause of the inappropriate examination of the subject is specified, but the instruction unit 27 can also instruct the user on how to change the breathing state of the subject, the pressing amount of the ultrasound probe 1, and the posture of the subject, which are specified as the causes of the inappropriate examination.

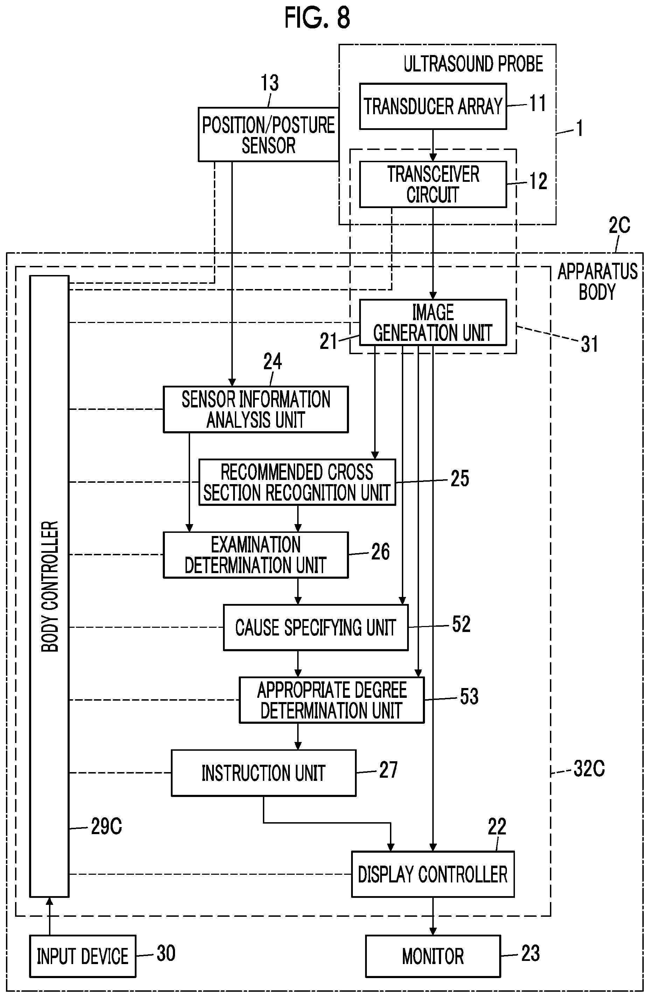

Fig. 8 shows a configuration of an ultrasound diagnostic apparatus according to Embodiment 4. The ultrasound diagnostic apparatus according to Embodiment 4 comprises an apparatus body 2C instead of the apparatus body 2B with respect to the ultrasound diagnostic apparatus according to Embodiment 3 shown in Fig. 7. The apparatus body 2C further comprises an appropriateness degree determination unit 53 in the apparatus body 2B according to Embodiment 3, and comprises a body controller 29C instead of the body controller 29B.

In the apparatus body 2C, the appropriateness degree determination unit 53 is connected to the image generation unit 21 and the cause specifying unit 52. The appropriateness degree determination unit 53 is connected to the instruction unit 27 and the body controller 29C. In addition, the image generation unit 21, the display controller 22, the sensor information analysis unit 24, the recommended cross section recognition unit 25, the examination determination unit 26, the instruction unit 27, the body controller 29C, the cause specifying unit 52, and the appropriateness degree determination unit 53 constitute a processor 32C for the apparatus body 2C.

The appropriateness degree determination unit 53 performs image analysis on the ultrasound image U generated by the image generation unit 21 to calculate at least one of a current level of the breathing state of the subject, a current level of the pressing amount of the ultrasound probe 1, or a current posture of the subject as an indicator related to the cause of the inappropriate examination of the subject specified by the cause specifying unit 52, and determines an appropriateness degree of the calculated indicator. Here, the appropriateness degree of the indicator is an evaluation values of the current level of the breathing state of the subject, the current level of the pressing amount of the ultrasound probe 1, and the current posture of the subject for the level of the breathing state of the subject, the level of the pressing amount of the ultrasound probe 1, and the posture of the subject, which can be determined to be appropriate for the examination of the subject, and represents how far the current state is from an appropriate state.

The appropriateness degree determination unit 53 can calculate the indicator by using, for example, a trained model in machine learning, which has learned a relationship between a large number of ultrasound images U in which it can be determined that the examination of the subject is inappropriate, the level of the breathing state of the subject, the level of the pressing amount of the ultrasound probe 1, and the posture of the subject.

The appropriateness degree determination unit 53 can calculate, for example, qualitative levels such as "breathing out deeply", "moderate", and "inhaling deeply" as the levels of the current breathing state of the subject, and can also calculate a numerical value representing the level of the breathing state. In addition, the appropriateness degree determination unit 53 can calculate, for example, qualitative levels such as "weak", "medium", and "strong" as the levels of the pressing amount of the current ultrasound probe 1, and can also calculate a numerical value indicating the pressing amount.

The appropriateness degree determination unit 53 can calculate the appropriateness degree based on the indicator by using, for example, a trained model in machine learning, which has learned a large amount of information on a relationship between the indicator related to the cause of the inappropriate examination of the subject and the appropriateness degree. The appropriateness degree determination unit 53 can calculate, for example, qualitative levels such as "a state of inhaling excessively compared to an appropriate breathing state", "a state of breathing out excessively compared to an appropriate breathing state", "less than an appropriate pressing amount", "more than an appropriate pressing amount", and "not an appropriate posture" as the appropriateness degrees, and can also calculate a quantitative level such as a difference from an appropriate breathing amount and a difference from an appropriate pressing amount.

The instruction unit 27 instructs the user on at least one of the amount of change in the level of the breathing state of the subject, the amount of change in the level of the pressing amount of the ultrasound probe 1, or the target posture of the subject, which is required to determine that the examination of the subject is appropriate, based on the indicator and the appropriateness degree that are obtained by the appropriateness degree determination unit 53.

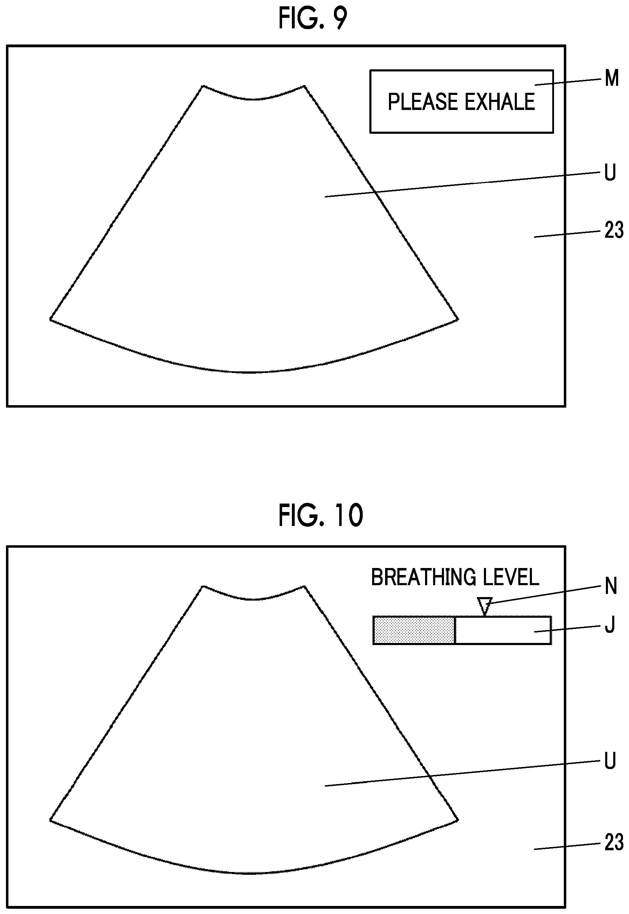

For example, in a case in which the appropriateness degree determined by the appropriateness degree determination unit 53 indicates that the subject is in a state of excessively exhaling compared to the appropriate breathing state, the instruction unit 27 can display the message M of "please inhale" on the monitor 23 as shown in Fig. 9. The instruction unit 27 can also display a message M, such as "Please exhale", "Please reduce the pressing amount of the probe", "Please increase the pressing amount of the probe", and "Please change the posture of the subject to ∘∘", on the monitor 23 with reference to the appropriateness degree. As described above, the instruction unit 27 can display the message M on the monitor 23 in accordance with the appropriateness degree.

In addition, the instruction unit 27 can also display, on the monitor 23, a relationship between a current indicator and an indicator required for determining that the examination is appropriate, based on the indicator obtained by the appropriateness degree determination unit 53 and the appropriateness degree. For example, as shown in Fig. 10, the instruction unit 27 can display a current breathing level of the subject and a breathing level required for determining that the examination is appropriate, on the monitor 23 by using the indicator J. A marker N indicating the breathing level required for determining that the examination is appropriate is given to the indicator J. Such an indicator J can also be used to indicate a relationship between a current level and an appropriate level with respect to the pressing amount of the ultrasound probe 1.

In addition, the instruction unit 27 can also indicate a relationship between the current level and the appropriate level by using a diagram other than the indicator J, and can also indicate a relationship between the current level and the appropriate level by using a numerical value.

As described above, with the ultrasound diagnostic apparatus according to Embodiment 4, the appropriateness degree determination unit 53 calculates at least one of the current level of the breathing state of the subject, the current level of the pressing amount of the ultrasound probe 1, or the current posture of the subject as the indicator related to the cause of the inappropriate examination of the subject, and determines the appropriateness degree of the calculated indicator, and the instruction unit 27 instructs the user on at least one of the amount of change in the level of the breathing state of the subject, the amount of change in the level of the pressing amount of the ultrasound probe 1, or the target posture of the subject, which is required to determine that the examination of the subject is appropriate, based on the indicator and the appropriateness degree, so that the user can appropriately visualize the recommended cross section by adjusting the breathing state of the subject, the pressing amount of the ultrasound probe 1, and the posture of the subject while checking at least one of the amount of change in the level of the breathing state of the subject, the amount of change in the level of the pressing amount of the ultrasound probe 1, or the target posture of the subject, which is instructed on by the instruction unit 27.

It should be noted that the description has been made in which the appropriateness degree determination unit 53 calculates the level of the breathing state of the subject by using a trained model in the machine learning. In general, it is known that the position of the abdomen or the chest rises in a case in which the subject exhales, and the position of the abdomen or the chest falls in a case in which the subject exhales. Therefore, for example, in a case in which the ultrasound diagnostic apparatus comprises a position sensor (not shown) attached to the body surface of the subject, such as the body surface of the abdomen or the body surface of the chest, the appropriateness degree determination unit 53 can also calculate the level of the breathing state of the subject based on the change in the height of the body surface of the subject detected by the position sensor. The position sensor can include, for example, at least one of an inertial sensor, a magnetic sensor, an optical sensor, or an optical camera.

In addition, although the description is made in which the appropriateness degree determination unit 53 calculates the level of the pressing amount of the ultrasound probe 1 by using a trained model in the machine learning, for example, in a case in which a pressure sensor (not shown) for measuring a pressing pressure of the ultrasound probe 1 against the subject is attached to the ultrasound probe 1, the appropriateness degree determination unit 53 can also calculate the level of the pressing amount of the ultrasound probe 1 based on the pressing pressure measured by the pressure sensor. In addition, for example, in a case in which the ultrasound diagnostic apparatus comprises a position sensor that detects a change in a height of the body surface of the subject pressed by the ultrasound probe 1, the level of the pressing amount of the ultrasound probe 1 can also be calculated based on the change in the height of the body surface of the subject detected by the position sensor. The position sensor can include, for example, at least one of an inertial sensor, a magnetic sensor, an optical sensor, or an optical camera.

### Explanation of References

1: ultrasound probe
2, 2A, 2B, 2C: apparatus body
11: transducer array
12: transceiver circuit
13: position/posture sensor
21: image generation unit
22: display controller
23: monitor
24: sensor information analysis unit
25: recommended cross section recognition unit
26: examination determination unit
27: instruction unit
29, 29A, 29B, 29C: body controller
30: input device
31: image acquisition unit
32, 32A, 32B, 32C: processor
41: pulser
42: amplifying unit
43: AD conversion unit
44: beam former
45: signal processing unit
46: DSC
47: image processing unit
51: position/posture detection unit
52: cause specifying unit
53: appropriateness degree determination unit
J: indicator
M: message
N: marker
U: ultrasound image

## Claims

1. An ultrasound diagnostic apparatus comprising:
an ultrasound probe (1);
a position/posture detection unit (51) configured to detect a position and a posture angle of the ultrasound probe (1);
an image acquisition unit (31) configured to acquire an ultrasound image of an inside of a subject by transmitting and receiving an ultrasound beam by using the ultrasound probe (1);
a recommended cross section recognition unit (25) configured to recognize a predetermined recommended cross section for a part of the subject by performing image analysis on the ultrasound image acquired by the image acquisition unit (31);
an examination determination unit (26) is configured to determine that an examination is inappropriate in a case in which
(i) the recommended cross section is not recognized by the recommended cross section recognition unit (25) after determining that the position and the posture angle of the ultrasound probe (1), which are detected by the position/posture detection unit (51), are in a predetermined positional/angular range including a recommended position and a recommended posture angle of the ultrasound probe (1) corresponding to the recommended cross section or
(ii) it is determined that the position and the posture angle of the ultrasound probe (1), which are detected by the position/posture detection unit (51), are in a predetermined positional/angular range including a recommended position and a recommended posture angle after determining that the recommended cross section is not recognized by the recommended cross section recognition unit (25); and
an instruction unit (27) configured to, in response to determining that an examination is inappropriate, instruct a user to change at least one of a breathing state of the subject, a pressing amount of the ultrasound probe (1) against the subject, or a posture of the subject in a case in which the examination determination unit (26) determines that the examination is inappropriate.

2. The ultrasound diagnostic apparatus according to claim 1, further comprising:
a cause specifying unit (52) configured to specify a cause of the examination being inappropriate from among the breathing state of the subject, the pressing amount of the ultrasound probe (1) against the subject, and the posture of the subject by performing the image analysis on the ultrasound image,
wherein the instruction unit (27) instructs the user to change at least one of the breathing state of the subject, the pressing amount of the ultrasound probe (1) against the subject, or the posture of the subject, which is specified as the cause by the cause specifying unit (52).

3. The ultrasound diagnostic apparatus according to claim 2, further comprising:
an appropriateness degree determination unit (53) configured to calculate at least one of a current level of the breathing state of the subject, a current level of the pressing amount of the ultrasound probe (1), or a current posture of the subject as an indicator related to the cause by performing the image analysis on the ultrasound image, and determines an appropriateness degree of the calculated indicator.

4. The ultrasound diagnostic apparatus according to claim 3,
wherein the instruction unit (27) instructs the user on at least one of an amount of change in the level of the breathing state, an amount of change in the level of the pressing amount of the ultrasound probe (1), or a target posture of the subject, which is required to determine that the examination is appropriate, based on the indicator and the appropriateness degree that are obtained by the appropriateness degree determination unit (53).

5. The ultrasound diagnostic apparatus according to any one of claims 1 to 4,
wherein the instruction unit (27) instructs the user to change at least one of the breathing state of the subject, the pressing amount of the ultrasound probe (1) against the subject, or the posture of the subject at an indicator position and an indicator posture angle of the ultrasound probe (1) for imaging an indicator cross section different from the recommended cross section.

6. A method of controlling an ultrasound diagnostic apparatus, the method comprising:
detecting (S1) a position and a posture angle of an ultrasound probe (1);
acquiring (S2) an ultrasound image of an inside of a subject by transmitting and receiving an ultrasound beam by using the ultrasound probe (1);
recognizing (S3) a predetermined recommended cross section for a part of the subject by performing image analysis on the ultrasound image;
determining (S5) that an examination is inappropriate in a case in which (i) the recommended cross section is not recognized by the recommended cross section recognition unit (25) after determining that the position and the posture angle of the ultrasound probe (1), which are detected by the position/posture detection unit (51), are in a predetermined positional/angular range including a recommended position and a recommended posture angle of the ultrasound probe (1) corresponding to the recommended cross section or (ii) it is determined that the position and the posture angle of the ultrasound probe (1), which are detected by the position/posture detection unit (51), are in a predetermined positional/angular range including a recommended position and a recommended posture angle after determining that the recommended cross section is not recognized by the recommended cross section recognition unit (25); and
instructing (S6) a user to change at least one of a breathing state of the subject, a pressing amount of the ultrasound probe (1) against the subject, or a posture of the subject in a case in which it is determined that the examination is inappropriate.

## Patentansprüche

1. Ultraschalldiagnosevorrichtung, umfassend:
eine Ultraschallsonde (1);
eine Positions-/Stellungsdetektionseinheit (51), die so konfiguriert ist, dass sie eine Position und einen Stellungswinkel der Ultraschallsonde (1) detektiert;
eine Bilderfassungseinheit (31), die so konfiguriert ist, dass sie ein Ultraschallbild eines Inneren einer Untersuchungsperson durch Übertragen und Empfangen eines Ultraschallstrahls durch Verwenden der Ultraschallsonde (1) erfasst;
eine Erkennungseinheit (25) für empfohlenen Querschnitt, die so konfiguriert ist, dass sie einen vorbestimmten empfohlenen Querschnitt für einen Teil der Untersuchungsperson durch Durchführen von Bildanalyse an dem von der Bilderfassungseinheit (31) erfassten Ultraschallbild erkennt;
eine Untersuchungsbestimmungseinheit (26), die so konfiguriert ist, dass sie bestimmt, dass eine Untersuchung in einem Fall ungeeignet ist, in dem
(i) der empfohlene Querschnitt von der Erkennungseinheit (25) für empfohlenen Querschnitt nicht erkannt wird, nachdem bestimmt worden ist, dass sich die Position und der Stellungswinkel der Ultraschallsonde (1), die von der Positions-/Stellungsdetektionseinheit (51) detektiert worden sind, in einem vorbestimmten Positions-/Winkelbereich befinden, der eine empfohlene Position und einen empfohlenen Stellungswinkel der Ultraschallsonde (1) enthält, die dem empfohlenen Querschnitt entsprechen, oder
(ii) bestimmt wird, dass sich die Position und der Stellungswinkel der Ultraschallsonde (1), die von der Positions-/Stellungsdetektionseinheit (51) detektiert worden sind, in einem vorbestimmten Positions-/Winkelbereich, der eine empfohlene Position und einen empfohlenen Stellungswinkel enthält, befinden, nachdem bestimmt worden ist, dass der empfohlene Querschnitt von der Erkennungseinheit (25) für empfohlenen Querschnitt nicht erkannt wird; und
eine Anweisungseinheit (27), die so konfiguriert ist, dass sie als Reaktion auf Bestimmen, dass eine Untersuchung ungeeignet ist, einen Benutzer in einem Fall, in dem die Untersuchungsbestimmungseinheit (26) bestimmt, dass die Untersuchung ungeeignet ist, anweist, mindestens eines von einem Atemzustand der Untersuchungsperson, einem Druckbetrag der Ultraschallsonde (1) gegen die Untersuchungsperson und einer Stellung der Untersuchungsperson zu ändern.

2. Ultraschalldiagnosevorrichtung nach Anspruch 1, ferner umfassend:
eine Ursachenspezifizierungseinheit (52), die so konfiguriert ist, dass sie eine Ursache dafür, dass die Untersuchung ungeeignet ist, aus dem Atemzustand der Untersuchungsperson, dem Druckbetrag der Ultraschallsonde (1) gegen die Untersuchungsperson und der Stellung der Untersuchungsperson durch Durchführen der Bildanalyse an dem Ultraschallbild spezifiziert,
wobei die Anweisungseinheit (27) den Benutzer anweist, mindestens eines von dem Atemzustand der Untersuchungsperson, dem Druckbetrag der Ultraschallsonde (1) gegen die Untersuchungsperson und der Stellung der Untersuchungsperson, das von der Ursachenspezifizierungseinheit (52) als die Ursache spezifiziert wird, zu ändern.

3. Ultraschalldiagnosevorrichtung nach Anspruch 2, ferner umfassend:
eine Angemessenheitsgrad-Bestimmungseinheit (53), die so konfiguriert ist, dass sie mindestens eines von einem aktuellen Niveau des Atemzustands der Untersuchungsperson, einem aktuellen Niveau des Druckbetrags der Ultraschallsonde (1) und einer aktuellen Stellung der Untersuchungsperson als einen Indikator, der sich auf die Ursache bezieht, durch Durchführen der Bildanalyse an dem Ultraschallbild berechnet und einen Angemessenheitsgrad des berechneten Indikators bestimmt.

4. Ultraschalldiagnosevorrichtung nach Anspruch 3,
wobei die Anweisungseinheit (27) den Benutzer über mindestens eines von einem Änderungsbetrag des Niveaus des Atemzustands, einem Änderungsbetrag des Niveaus des Druckbetrags der Ultraschallsonde (1) und einer Zielstellung der Untersuchungsperson, das erforderlich ist, um zu bestimmen, dass die Untersuchung angemessen ist, auf der Grundlage des Indikators und des Angemessenheitsgrads, die von der Angemessenheitsgrad-Bestimmungseinheit (53) erhalten werden, anweist.

5. Ultraschalldiagnosevorrichtung nach einem der Ansprüche 1 bis 4,
wobei die Anweisungseinheit (27) den Benutzer anweist, mindestens eines von dem Atemzustand der Untersuchungsperson, dem Druckbetrag der Ultraschallsonde (1) gegen die Untersuchungsperson und der Stellung der Untersuchungsperson bei einer Indikatorposition und einem Indikatorstellungswinkel der Ultraschallsonde (1) zum Abbilden eines Indikatorquerschnitts, der sich von dem empfohlenen Querschnitt unterscheidet, zu ändern.

6. Verfahren des Steuerns einer Ultraschalldiagnosevorrichtung, wobei das Verfahren umfasst:
Detektieren (S1) einer Position und eines Stellungswinkels einer Ultraschallsonde (1);
Erfassen (S2) eines Ultraschallbildes eines Inneren einer Untersuchungsperson durch Übertragen und Empfangen eines Ultraschallstrahls durch Verwenden der Ultraschallsonde (1);
Erkennen (S3) eines vorbestimmten empfohlenen Querschnitts für einen Teil der Untersuchungsperson durch Durchführen von Bildanalyse an dem Ultraschallbild;
Bestimmen (S5), dass eine Untersuchung in einem Fall ungeeignet, in dem (i) der empfohlene Querschnitt von der Erkennungseinheit (25) für empfohlenen Querschnitt nicht erkannt wird, nachdem bestimmt worden ist, dass sich die Position und der Stellungswinkel der Ultraschallsonde (1), die von der Positions-/Stellungsdetektionseinheit (51) detektiert worden sind, in einem vorbestimmten Positions-/Winkelbereich befinden, der eine empfohlene Position und einen empfohlenen Stellungswinkel der Ultraschallsonde (1) enthält, die dem empfohlenen Querschnitt entsprechen, oder (ii) bestimmt wird, dass sich die Position und der Stellungswinkel der Ultraschallsonde (1), die von der Positions-/Stellungsdetektionseinheit (51) detektiert worden sind, in einem vorbestimmten Positions-/Winkelbereich, der eine empfohlene Position und einen empfohlenen Stellungswinkel enthält, befinden, nachdem bestimmt worden ist, dass der empfohlene Querschnitt von der Erkennungseinheit (25) für empfohlenen Querschnitt nicht erkannt wird; und
Anweisen (S6) eines Benutzers, mindestens eines von einem Atemzustand der Untersuchungsperson, einem Druckbetrag der Ultraschallsonde (1) gegen die Untersuchungsperson und einer Stellung der Untersuchungsperson zu ändern, in einem Fall, in dem bestimmt wird, dass die Untersuchung ungeeignet ist.

## Revendications

1. Appareil de diagnostic par ultrasons comprenant :
une sonde ultrasonore (1) ;
une unité de détection de position/posture (51) configurée pour détecter une position et un angle de posture de la sonde ultrasonore (1) ;
une unité d'acquisition d'images (31) configurée pour acquérir une image ultrasonore d'un intérieur d'un sujet en transmettant et en recevant un faisceau ultrasonore en utilisant la sonde ultrasonore (1) ;
une unité de reconnaissance de section transversale recommandée (25) configurée pour reconnaître une section transversale recommandée prédéterminée pour une partie du sujet en effectuant une analyse d'image sur l'image ultrasonore acquise par l'unité d'acquisition d'images (31) ;
une unité de détermination d'examen (26) configurée pour déterminer qu'un examen est inapproprié dans un cas où
(i) la section transversale recommandée n'est pas reconnue par l'unité de reconnaissance de section transversale recommandée (25) après avoir déterminé que la position et l'angle de posture de la sonde ultrasonore (1), qui sont détectés par l'unité de détection de position/posture (51), se trouvent dans une plage positionnelle/angulaire prédéterminée incluant une position recommandée et un angle de posture recommandé de la sonde ultrasonore (1) correspondant à la section transversale recommandée ou
(ii) il est déterminé que la position et l'angle de posture de la sonde ultrasonore (1), qui sont détectés par l'unité de détection de position/posture (51), se trouvent dans une plage positionnelle/angulaire prédéterminée incluant une position recommandée et un angle de posture recommandé après avoir déterminé que la section transversale recommandée n'est pas reconnue par l'unité de reconnaissance de section transversale recommandée (25) ; et
une unité d'instruction (27) configurée pour, en réponse à la détermination qu'un examen est inapproprié, instruire un utilisateur de modifier au moins l'un parmi un état respiratoire du sujet, une force de pression de la sonde ultrasonore (1) contre le sujet ou une posture du sujet dans un cas où l'unité de détermination d'examen (26) détermine que l'examen est inapproprié.

2. Appareil de diagnostic par ultrasons selon la revendication 1, comprenant en outre :
une unité de spécification de cause (52) configurée pour spécifier une cause de l'examen inapproprié parmi l'état respiratoire du sujet, la force de pression de la sonde ultrasonore (1) contre le sujet et la posture du sujet en effectuant l'analyse d'image sur l'image ultrasonore,
dans lequel l'unité d'instruction (27) instruit l'utilisateur de modifier au moins l'un parmi l'état respiratoire du sujet, la force de pression de la sonde ultrasonore (1) contre le sujet ou la posture du sujet, qui est spécifié comme la cause par l'unité de spécification de cause (52).

3. Appareil de diagnostic par ultrasons selon la revendication 2, comprenant en outre :
une unité de détermination de degré d'adéquation (53) configurée pour calculer au moins l'un parmi un niveau actuel de l'état respiratoire du sujet, un niveau actuel de la force de pression de la sonde ultrasonore (1) ou une posture actuelle du sujet comme un indicateur relatif à la cause en effectuant l'analyse d'image sur l'image ultrasonore, et pour déterminer un degré d'adéquation de l'indicateur calculé.

4. Appareil de diagnostic par ultrasons selon la revendication 3,
dans lequel l'unité d'instruction (27) instruit l'utilisateur sur au moins l'un parmi une quantité de changement dans le niveau de l'état respiratoire, une quantité de changement dans le niveau de la force de pression de la sonde ultrasonore (1) ou une posture cible du sujet, qui est nécessaire pour déterminer que l'examen est approprié, sur la base de l'indicateur et du degré d'adéquation qui sont obtenus par l'unité de détermination de degré d'adéquation (53).

5. Appareil de diagnostic par ultrasons selon l'une quelconque des revendications 1 à 4,
dans lequel l'unité d'instruction (27) instruit l'utilisateur de modifier au moins l'un parmi l'état respiratoire du sujet, la force de pression de la sonde ultrasonore (1) contre le sujet ou la posture du sujet à une position d'indicateur et à un angle de posture d'indicateur de la sonde ultrasonore (1) pour imager une section transversale d'indicateur différente de la section transversale recommandée.

6. Procédé de contrôle d'un appareil de diagnostic par ultrasons, le procédé comprenant :
détecter (S1) une position et un angle de posture d'une sonde ultrasonore (1) ;
acquérir (S2) une image ultrasonore d'un intérieur d'un sujet en transmettant et en recevant un faisceau ultrasonore en utilisant la sonde ultrasonore (1) ;
reconnaître (S3) une section transversale recommandée prédéterminée pour une partie du sujet en effectuant une analyse d'image sur l'image ultrasonore ;
déterminer (S5) qu'un examen est inapproprié dans un cas où (i) la section transversale recommandée n'est pas reconnue par l'unité de reconnaissance de section transversale recommandée (25) après avoir déterminé que la position et l'angle de posture de la sonde ultrasonore (1), qui sont détectés par l'unité de détection de position/posture (51), se trouvent dans une plage positionnelle/angulaire prédéterminée incluant une position recommandée et un angle de posture recommandé de la sonde ultrasonore (1) correspondant à la section transversale recommandée ou (ii) il est déterminé que la position et l'angle de posture de la sonde ultrasonore (1), qui sont détectés par l'unité de détection de position/posture (51), se trouvent dans une plage positionnelle/angulaire prédéterminée incluant une position recommandée et un angle de posture recommandé après avoir déterminé que la section transversale recommandée n'est pas reconnue par l'unité de reconnaissance de section transversale recommandée (25) ; et
instruire (S6) un utilisateur de modifier au moins l'un parmi un état respiratoire du sujet, une force de pression de la sonde ultrasonore (1) contre le sujet ou une posture du sujet dans un cas où il est déterminé que l'examen est inapproprié.
